# EUROPEAN PATENT APPLICATION

(11) **EP 4 053 142 A1**
(43) Date of publication of application: **07.09.2022**
(21) Application number: 20881941.7
(22) Date of filing: 22.10.2020
(51) Int. Cl.: C07H 17/07, A61Q 19/06, A61K 8/60

(54) **EXTERNAL SKIN PREPARATION FOR AMELIORATING CELLULITE AND METHOD FOR AMELIORATING CELLULITE**

(30) Priority: 30.10.2019 JP 2019197581
(71) Applicant: Toyo Sugar Refining Co., Ltd., Tokyo 103-0016 (JP)
(72) Inventor: HASHIZUME Yushi, Ichihara-shi, Chiba 290-0046 (JP); TANDIA Mahamadou, Noda-shi, Chiba 278-0027 (JP)
(74) Representative: Dehns
(86) International application number: PCT/JP2020/039687
(87) International publication number: WO 2021/085293

(57) **Abstract**

An objective of the present invention is to provide a skin external preparation for cellulite improvement and a cellulite improvement method that do not require any special equipment and manipulation and are capable of conveniently improving cellulite. The skin external preparation for cellulite improvement containing at least one component (A) selected from α-glucosyl hesperidin and α-glucosyl naringin. The cellulite improvement method including a step of externally applying the skin external preparation for cellulite improvement containing at least one component (A) selected from α-glucosyl hesperidin and α-glucosyl naringin to the skin of a target.

## Description

### Technical Field

The present invention relates to a skin external preparation for cellulite improvement and a cellulite improvement method.

### Background Art

Cellulite refers to the state of skin exhibiting an uneven appearance and is also called orange peel due to its appearance that looks like the external skin of oranges. Cellulite is likely to be formed in the thighs and hips abundantly containing subcutaneous fat and is thus one of cosmetic troubles experienced by women.

The subcutaneous fatty tissue is largely composed of fat cells, and a clump of fat cells forms a lobulated structure that is called a fat lobule. In addition, bundles of collagen fibers formed from dermis run through and occur in the subcutaneous fatty tissue here and there and strongly connect the dermis to the fascia and the periostea. The cause of the formation of cellulite is still largely unclear, but is considered that fat lobules are enlarged or the structure and state of connective tissues change, which makes the bundles of collagen fibers pull the skin and makes denatured fat tissues appear unevenly on skin surfaces (Non Patent Literature 1).

In recent years, ultrasonic tomography has been applied for the observation of the skin, in which fat cells and lymph, which are anechoic structures, appear black and connective tissues, which are hyper echoic, appear green, red or yellow depending on echo levels. It has been reported that, when the images of subjects are compared by age group, the images of the subjects in their twenties contain many red portions and yellow portions and, as the subjects are older, the ratio of green portions and black portions increase (Non Patent Literature 1). That is, as the subjects are older, echoes from connective tissues become weaker, and the amount and state of collagen in the dermis change more. In ultrasonic tomography, since the echo levels are significantly different in the dermis composed of connective tissues and subcutaneous tissue including many fat cells, the boundary surface between dermis and subcutaneous tissue is clear. In ultrasonic tomographic images of normal skin with no cellulite, the boundary surface between the dermis and the subcutaneous tissue appears smooth and linear. On the other hand, for the skin with cellulite, the boundary surface between the dermis and the subcutaneous tissue appears irregular and disordered (Non Patent Literature 1 and 2). Therefore, the observation of the boundary surface between the dermis and the subcutaneous by ultrasonic tomography makes it possible to evaluate the presence or absence and state of cellulite.

For cellulite improvement, skin massage is considered to work well, for example, but massage needs to be done by skilled aestheticians, and also there are cases where the effect of massage is not guaranteed. Regarding therapies such as ultrasonic therapy and massage therapy with equipment, there have been some cases in which the effects thereof have been reported by ultrasonic tomography (Non Patent Literatures 1 and 2); however, these therapies required special equipment. In addition, a cellulite inhibitor containing fullerene as an active ingredient (Patent Literature 1) is known, but this cellulite inhibitor simply has an effect of preventing intracellular fat droplet accumulation, and there has been no report regarding an effect of improving cellulite that had already been formed.

### Citation List

### Patent Literature

Patent Literature 1
International Publication No. WO 2006/011644

### Non Patent Literature

Non Patent Literature 1 C. Christ, R., Improvement in skin elasticity in the treatment of cellulite and connective tissue weakness by means of Extracorporeal Pulse Activation Therapy: EPATR., Aesthetic Surgery Journal, 2008, Vol. 28, Number 5, 538 to 544
Non Patent Literature 2 Lucassen G. W., The effectiveness of massage treatment on cellulite as monitored by ultrasound imaging., Skin Research and Technology, 1997, Vol. 3, 154 to 160

### Summary of Invention

### Technical Problem

An objective of the present invention is to provide a skin external preparation for cellulite improvement and a cellulite improvement method that do not require any special equipment and manipulation and are capable of conveniently improving cellulite.

### Solution to Problem

The present inventors carried out intensive studies to solve the above-described problems. As a result, the present inventors have found that the above-described problems can be solved by the following configurations and completed the present invention. The present invention provides, for example, the following [1] to [7].

[1] A skin external preparation for cellulite improvement, containing at least one component (A) selected from α-glucosyl hesperidin and α-glucosyl naringin.
[2] The skin external preparation for cellulite improvement according to [1], being for massage.
[3] The skin external preparation for cellulite improvement according to [1] or [2], in which a content of the component (A) is 0.01 to 10 mass%.
[4] A cellulite improvement method, including a step of externally applying the skin external preparation for cellulite improvement according to any one of [1] to [3] to the skin of a target.
[5] The cellulite improvement method according to [4], further including a step of massaging the skin in at least one timing of before the step of externally applying the skin external preparation for cellulite improvement for the skin, at the same time as the step of externally applying the skin external preparation for cellulite improvement for the skin and after the step of externally applying the skin external preparation for cellulite improvement for the skin.
[6] The cellulite improvement method according to [4] or [5], in which the component (A) is administered at 0.001 to 10 mg/kg body weight per day.
[7] The cellulite improvement method according to any one of [4] to [6], further having a step of evaluating the cellulite of a target by ultrasonic tomography.

### Advantageous Effect of Invention

According to the present invention, it is possible to provide a skin external preparation for cellulite improvement and a cellulite improvement method that do not require any special equipment and manipulation and are capable of conveniently improving cellulite.

### Brief Description of Drawings

[Figure 1] Figure 1 is an ultrasonic tomographic image of the back side of a thigh of subject 1 before application of α-glucosyl hesperidin-blended massage cream to the subject, after 4 weeks of the application and after 8 weeks of the application.
[Figure 2] Figure 2 is an ultrasonic tomographic image of the back side of the thigh of subject 1 before application of α-glucosyl naringin-blended massage cream to the subject, after 4 weeks of the application and after 8 weeks of the application.
[Figure 3] Figure 3 is an ultrasonic tomographic image of the back side of a thigh of subject 2 before application of the α-glucosyl hesperidin-blended massage cream to the subject, after 4 weeks of the application and after 8 weeks of the application.
[Figure 4] Figure 4 is an ultrasonic tomographic image of the back side of the thigh of subject 2 before application of the α-glucosyl naringin-blended massage cream to the subject, after 4 weeks of the application and after 8 weeks of the application.
[Figure 5] Figure 5 is an ultrasonic tomographic image of the back side of a thigh of subject 3 before application of the α-glucosyl hesperidin-blended massage cream to the subject, after 4 weeks of the application and after 8 weeks of the application.
[Figure 6] Figure 6 is an ultrasonic tomographic image of the back side of the thigh of subject 3 before application of the α-glucosyl naringin-blended massage cream to the subject, after 4 weeks of the application and after 8 weeks of the application.
[Figure 7] Figure 7 is a graph showing a change in cellulite area among before the application of the α-glucosyl hesperidin-blended massage cream, after 4 weeks of the application and after 8 weeks of the application.
[Figure 8] Figure 8 is a graph showing a change in collagen score among before the application of the α-glucosyl hesperidin-blended massage cream, after 4 weeks of the application and after 8 weeks of the application.
[Figure 9] Figure 9 is a graph showing a change in age band among before the application of the α-glucosyl hesperidin-blended massage cream, after 4 weeks of the application and after 8 weeks of the application.
[Figure 10] Figure 10 is a graph showing a change in thickness among before the application of the α-glucosyl hesperidin-blended massage cream, after 4 weeks of the application and after 8 weeks of the application.
[Figure 11] Figure 11 is a graph showing a change in cellulite area among before the application of the α-glucosyl naringin-blended massage cream, after 4 weeks of the application and after 8 weeks of the application.
[Figure 12] Figure 12 is a graph showing a change in collagen score among before the application of the α-glucosyl naringin-blended massage cream, after 4 weeks of the application and after 8 weeks of the application.
[Figure 13] Figure 13 is a graph showing a change in age band among before the application of the α-glucosyl naringin-blended massage cream, after 4 weeks of the application and after 8 weeks of the application.
[Figure 14] Figure 14 is a graph showing a change in thickness among before the application of the α-glucosyl naringin-blended massage cream, after 4 weeks of the application and after 8 weeks of the application.

### Description of Embodiment

Next, a skin external preparation for cellulite improvement and a cellulite improvement method of the present invention will be specifically described. The skin external preparation for cellulite improvement of the present invention contains at least one component (A) selected from α-glucosyl hesperidin and α-glucosyl naringin.

### <α-Glucosyl Hesperidin>

α-Glucosyl hesperidin (also referred to as α-glucosyl hesperidin or αG hesperidin) is a collective term of compounds in which one or more molecules of glucose are added to a hydroxyl group in the rutinose unit of hesperidin by an α-1,4 linkage. α-Glucosyl hesperidin in the present invention may be composed only of one compound having such a structure or may be a mixture of two or more compounds having such a structure. Hesperidin is a compound in which β-rutinose (6-O-α-L-rhamnosyl-β-D-glucose) is linked to a hydroxyl group at the position 7 of hesperetin, that is, a hesperetin glycoside.

α-Glucosyl hesperidin can be represented by the following formula (1). In the formula (1), n is 0 or an integer of 1 or more. n is preferably an integer of 0 to 19, more preferably an integer of 0 to 10, still more preferably an integer of 0 to 5 and particularly preferably 0.

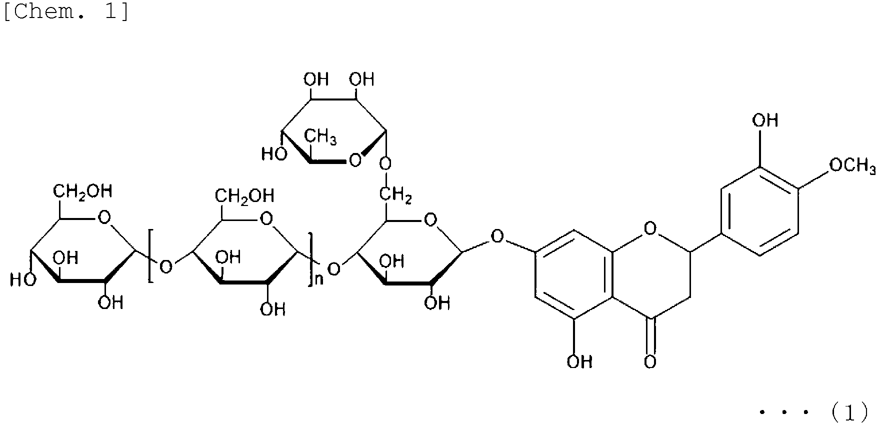

α-Glucosyl hesperidin is a compound that is contained as a main component in a mixture known as "enzyme-treated hesperidin" (also called "transglycosylated hesperidin" in some cases). The enzyme-treated hesperidin is a mixture of compounds formed by enzyme treatment of sugar of hesperidin, contains α-glucosyl hesperidin (including monoglucosyl hesperidin) and may further contain a hesperidin derivative other than α-glucosyl hesperidin such as 7-glucosyl hesperetin (also referred to as the different hesperidin derivative). However, the enzyme-treated hesperidin preferably does not contain hesperetin.

Examples of the enzyme treatment of the sugar of hesperidin are as described below. (1) A glycosyltransferase is caused to act on hesperidin in the coexistence of a glycosyl donor to add glucose to the glucose unit of hesperidin by an α-1,4 linkage, whereby α-glucosyl hesperidin is formed, and a composition containing unreacted hesperidin and α-glucosyl hesperidin is obtained (first enzyme-treated hesperidin). (2) Glucoamylase, for example, is caused to act on the α-glucosyl hesperidin formed by (1), and all glucose molecules other than one molecule from glucose chains linked to the glucose unit of hesperidin are cut, whereby α-monoglucosyl hesperidin is formed, and a composition containing unreacted hesperidin and α-monoglucosyl hesperidin is obtained (second enzyme-treated hesperidin). (3) α-L-rhamnosidase is caused to act on the unreacted hesperidin in (2), rhamnose that is contained in the rutinose unit of rutin is cut to form 7-glucosyl hesperetin, and a composition containing 7-glucosyl hesperetin and α-monoglucosyl hesperidin is obtained (third enzyme-treated hesperidin). In the skin external preparation for cellulite improvement or the cellulite improvement method in the present invention, a composition containing α-glucosyl hesperidin of any of the first enzyme-treated hesperidin, the second enzyme-treated hesperidin and the third enzyme-treated hesperidin may be used.

Examples of the first enzyme treatment include treatment in which a glycosyltransferase (for example, an enzyme having a function of adding glucose to hesperidin such as cyclodextrin glucanotransferase (CGTase, EC2.4.1.19)) is caused to act on hesperidin in the coexistence of an α-glucosyl sugar compound (for example, cyclodextrin, partially degraded starch).

Normally, the enzyme-treated hesperidin contains a mixture of compounds in which the number of glucoses linked to hesperidin is different from one another, for example, a mixture composed of monoglucosyl hesperidin and different α-glucosyl hesperidin. In addition, the enzyme-treated hesperidin is ordinarily produced by enzyme treatment and thus contains unreacted hesperidin or a different hesperidin derivative, for example, 7-glucosyl hesperetin in some cases.

When the effect of the present invention is taken into account, the enzyme-treated hesperidin is preferably a mixture containing at least α-glucosyl hesperidin and further containing any one or both of hesperidin and 7-glucosyl hesperetin.

Examples of the commercially available product of the enzyme-treated hesperidin include a product "αG hesperidin PS-CC" manufactured by Toyo Sugar Refining Co., Ltd. The "αG hesperidin PS-CC" contains 80 mass% or more of monoglucosyl hesperidin and contains hesperidin and 7-glucosyl hesperetin.

As the α-glucosyl hesperidin, α-monoglucosyl hesperidin to which one molecule of α-glucose is linked is preferable. In the present invention, enzyme-treated hesperidin containing 70 mass% or more of α-monoglucosyl hesperidin based on the entire enzyme-treated hesperidin (composition) is preferably used.

The α-monoglucosyl hesperidin can be produced by causing a glycohydrolase to act on α-glucosyl hesperidin to which two or more molecules of glucose are linked and cutting the glucose linked to hesperidin except only one glucose molecule (second enzyme-treated hesperidin). As the glycohydrolase, an enzyme having a glucoamylase activity that cuts an α-1,4-glucoside linkage by glucose unit, for example, glucoamylase (EC3.2.1.3) is exemplified. The proportion of the monoglucosyl hesperidin in the α-glucosyl hesperidin can be adjusted according to conditions such as the temperature and time conditions of the enzyme treatment using glucoamylase, and furthermore, a method in which monoglucosyl hesperidin is purified and collected from the mixture as the enzyme-treated hesperidin is also well known.

The presence of α-glucosyl hesperidin, hesperidin and other components that are contained in the enzyme-treated hesperidin can be confirmed by HPLC chromatogram, and the contents of the individual components and the purity of a desired specific component can be calculated from the peak area of the chromatogram.

The method for producing α-glucosyl hesperidin is not particularly limited, and a well-known method can be used. α-Glucosyl hesperidin is preferably produced by the above-described enzyme treatment of hesperidin since the yield is favorable and the production is easy. The method for procuring and preparing hesperidin is not particularly limited, and a reagent or a compound that is ordinarily produced and sold as a purified substance may be used or a compound prepared by extraction from a raw material such as the external skins of citrus fruits (such as tangerines and oranges) may be used.

### <α-Glucosyl Naringin>

α-Glucosyl naringin (also referred to as α-glucosyl naringin or αG naringin) is a collective term of compounds in which one or more molecules of glucose are added to a hydroxyl group in naringin. Naringin is one of flavonoids having a structure in which neohesperidose (L-rhamnosyl-(α1→2)-D-glucose) is β-linked to a hydroxyl group at the position 7 of a naringenin (5,7,4'-trihydroxyflavanone) skeleton. α-Glucosyl naringin has a structure in which one or more molecules of α-glucose are linked to at least one of a hydroxyl group at the position 3 (position 3") of a glucose residue in a rutinose residue and a hydroxyl group at the position 4 (position 4') of a phenyl group in a naringenin skeleton, which are in the naringin. α-Glucosyl naringin in the present invention may be composed only of one compound having such a structure or may be a mixture of two or more compounds having such a structure.

α-Glucosyl naringin can be represented by the following formula [I]. In the formula [I], m in R¹ and n in R² each mean the number of α-glucose residues linked to the position 3" or the position 4' and each independently represent an integer of 0 or more and, normally, 25 or less. Here, in order for the formula [I] to represent "α-glucosyl naringin", it is necessary that m + n ≥ 1 is satisfied, that is, at least one molecule of α-glucose is linked to naringin (in the case of m = n = 0, that is, in a case where both R¹ and R² are -OH, the formula [I] represents "naringin").

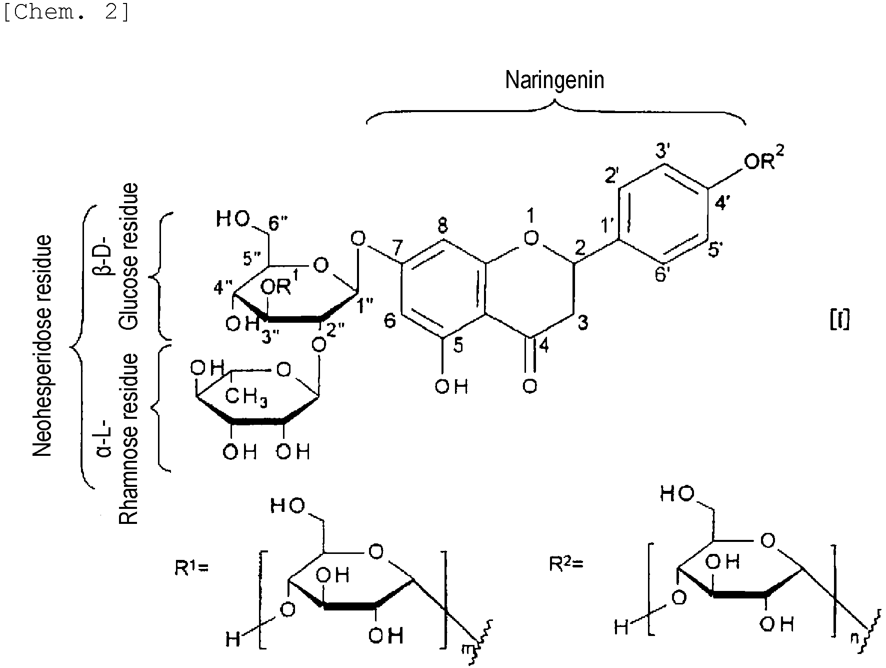

α-Glucosyl naringin is a compound that is contained as a main component in a material known as "enzyme-treated naringin" (also called "transglucosylated naringin" in some cases). The enzyme-treated naringin is a product that is obtained by reacting a glycosyltransferase (for example, cyclodextrin glycosyltransferase) with a mixture of naringin and a glycosyl donor (for example, dextrin) (called as "first enzyme-treated naringin" in the present specification) and is a mixture of a variety of compounds in which one or more molecules of glucose are added to a hydroxyl group in naringin, that is, a mixture containing α-glucosyl naringin and naringin that is an unreacted substance. Regarding the basic method for producing the first enzyme-treated naringin, for example, Japanese Unexamined Patent Application Publication No.H4-13691 can be referred to. If necessary, the first enzyme-treated naringin is purified using, for example, a porous synthetic adsorbent and an appropriate eluent, whereby a first enzyme-treated naringin in which the glycosyl donor and other impurities are removed, furthermore, the content of naringin is decreased and the purity of α-glucosyl naringin is increased (α-glucosyl naringin purified substance) can be obtained.

As α-glucosyl naringin, a substance in which one molecule of α-glucose is linked to the position 3" of naringin and/or one molecule of α-glucose is linked to the position 4' of naringin, that is, 3"-α-monoglucosyl naringin (in the formula [1], m = 1 and n = 0), 4'-α-monoglucosyl naringin (in the formula [1], m = 0 and n = 1) or 3"-4'-α-diglucosyl naringin (in the formula [1], m = 1 and n = 1) is preferable, and, particularly, 3"-α-monoglucosyl naringin is more preferable. In the present invention, enzyme-treated naringin containing 80 wt% or more of α-monoglucosyl naringin, particularly, 3"-α-monoglucosyl naringin based on the entire enzyme-treated naringin (composition) is preferably used.

Enzyme-treated naringin containing the above-described three kinds of α-mono/diglucosyl naringin more than the first enzyme-treated naringin (called as "second enzyme-treated naringin" in the present specification) can be obtained by, for example, treating the above-described first enzyme-treated naringin with an enzyme having a glucoamylase activity and cutting sugar chains which have been transferred to the position 3" and/or the position 4' of naringin by the glycosyltransferase and to which two or more molecules of α-glucose are linked by α-1,4 linkages except only α-glucose residues as many as one molecule of the base. Furthermore, the second enzyme-treated naringin is treated with an enzyme having an α-glucosidase activity, and α-glucose residues as many as one molecule directly linked to the hydroxyl group at the position 4' are cut, whereby an enzyme-treated naringin in which 3"-α-monoglucosyl naringin is left and 4'-α-monoglucosyl naringin and 3"-4'-α-diglucosyl naringin are rarely or not contained (called as "third enzyme-treated naringin" in the present specification) can be obtained. Regarding the basic method for producing the second and third enzyme-treated naringin, for example, Japanese Unexamined Patent Application Publication No.2002-199896 can be referred to.

In addition, when transglucosidase is caused to act on the first enzyme-treated naringin, since transglucosidase is an enzyme having both a glucoamylase activity and an α-glucodisase activity, the third enzyme-treated naringin containing abundant 3"-α-monoglucosyl naringin can be obtained in one step rather than two-step treatment as described above. Furthermore, if necessary, treatment in which an enzyme having a β-glucodidase activity is caused to act on the third enzyme-treated naringin (may be caused to act on the first enzyme-treated naringin simultaneously with transglucosidase) to cut a neohesperidose residue which is in unreacted naringin that has been dissolved in an aqueous solution in a small amount (and in which a sugar chain has not been modified by the glycosyltransferase) from naringenin, which is an aglycone of the neohesperidose residue may be carried out. Naringenin that is formed by such treatment has a lower solubility than naringin and is thus precipitated and can be easily removed from the aqueous solution, which makes it possible to collect 3"-α-monoglucosyl naringin from the aqueous solution with a higher purity.

The presence of α-glucosyl naringin, naringin and other components that are contained in the enzyme-treated naringin can be confirmed by HPLC chromatogram, and the contents of the individual components and the purity of a desired specific component can be calculated from the peak area of the chromatogram.

The method for producing α-glucosyl naringin is not particularly limited, and a well-known method can be used. α-Glucosyl naringin is preferably produced by the above-described enzyme treatment of naringin since the yield is favorable and the production is easy. The method for procuring and preparing naringin is not particularly limited, and a reagent or a compound that is ordinarily produced and sold as a purified substance may be used or a compound prepared by extraction from a raw material such as the external skins of citrus fruits (such as Chinese citrons and grapefruits) may be used.

### <Cellulite Improvement>

Cellulite improvement refers to the obtainment of a more favorable evaluation than ever in cellulite evaluation. The method for evaluating cellulite is not particularly limited and may be appearance evaluation using equipment, tomography, histochemical evaluation by skin specimen collection, visual appearance evaluation, questionnaire survey from subjects or evaluators, or determination by users, for example. Since the operation is convenient and objective results can be obtained, it is preferable that cellulite improvement in the skin external preparation for cellulite improvement of the present invention can be confirmed in the evaluation using equipment, evaluation by tomography is more preferable, and evaluation by ultrasonic tomography is still more preferable.

Equipment used to carry out ultrasonic tomography is not particularly limited, and well-known equipment can be used. Examples thereof include DermaLab manufactured by Cortex Technology.

The index of cellulite improvement is not particularly limited, but a change in characteristic structure, shape or state of skin containing cellulite, such as unevenness on the skin surface, is preferably used as the index, and a change in characteristic structure, shape or state of skin containing cellulite, which is observed at the time of analyzing skin in the depth direction, is more preferably used as the index. Examples of the characteristic structure, shape or state of skin containing cellulite, which is observed at the time of analyzing skin in the depth direction, include the enlargement of fat lobules, a change in structure, shape or state of connective tissues, the shape of the boundary surface between dermis and subcutaneous tissue, preferably a wavy shape of the boundary surface between dermis and subcutaneous tissue, a decrease in density of collagen in dermis, the width of skin with less collagen or the thickness of a collagen layer, and more preferably a wavy shape of the boundary surface between dermis and subcutaneous tissue. The wavy shape of the boundary surface between dermis and subcutaneous tissue may be evaluated from the length of the boundary surface per a certain length or area of skin. In addition, the wavy shape of the boundary surface between dermis and subcutaneous tissue may be evaluated by, as described in Examples, setting a reference surface parallel to the skin surface at a deeper part in the skin than the boundary surface between dermis and subcutaneous tissue and calculating the volume of a space between the reference surface and the boundary surface between dermis and subcutaneous tissue or the area obtained by cutting the volume by a specific surface. In a case where cellulite improvement is evaluated using DermaLab manufactured by Cortex Technology, a decrease in cellulite area, an increase in collagen score, a decrease in age band or a decrease in thickness of a dermis collagen layer is preferably used as the index.

A target site of the skin external preparation for cellulite improvement or the cellulite improvement method of the present invention is not particularly limited as long as cellulite is observed in the site, but is preferably a thigh, a hip or an abdominal area where cellulite appears more commonly and more preferably a thigh or a hip, and still more preferably the back side of a thigh.

The period of time during which the skin external preparation for cellulite improvement or the cellulite improvement method of the present invention is applied is not particularly limited, but is preferably one week to 12 months, more preferably two weeks to six months and still more preferably three weeks to three months since a cellulite improvement effect is effectively developed.

The number of times of applying the skin external preparation for cellulite improvement or the cellulite improvement method of the present invention is not particularly limited, but is preferably once to three times per day, more preferably once or twice per day and still more preferably twice per day since the cellulite improvement effect is effectively developed.

### <Skin External Preparation for Cellulite Improvement>

The skin external preparation for cellulite improvement of the present invention needs to contain at least one component (A) selected from α-glucosyl hesperidin and α-glucosyl naringin. As the component (A), the skin external preparation for cellulite improvement may contain α-glucosyl hesperidin or α-glucosyl naringin, or may contain both α-glucosyl hesperidin and α-glucosyl naringin. The skin external preparation for cellulite improvement preferably contains α-glucosyl hesperidin or α-glucosyl naringin as the component (A) since the cellulite improvement effect is effectively developed.

The skin external preparation for cellulite improvement of the present invention may be composed only of the component (A) or may be a preparation in which the component (A) is blended with a pharmaceutically acceptable carrier or a variety of additives, for example, as long as the effect of the present invention is not hindered.

The skin external preparation for cellulite improvement can be produced as a medicine, a quasi-drug or a cosmetic product. In order to effectively develop the cellulite improvement effect, the skin external preparation for cellulite improvement is preferably for massage use.

The form and dosage form of the skin external preparation for cellulite improvement of the present invention are not particularly limited and are normally a liquid form (including a paste form and a gel form). Specific examples thereof include a liquid external preparation, suspension, emulsion, cream and ointment. These dosage forms or other dosage forms are appropriately selected depending on the purposes. Among them, a liquid external preparation, cream or ointment is preferable and a liquid external preparation, cream or ointment for massage use is more preferable since the administration is easy and the cellulite improvement effect is easily developed.

The skin external preparation for cellulite improvement can be produced by adding α-glucosyl hesperidin or α-glucosyl naringin according to a method that is ordinarily used for these preparations. α-Glucosyl hesperidin or α-glucosyl naringin may be added in the initial stage of the production step of the preparation or may be added in the middle stage or final stage of the production step, and, as the addition method, an appropriate method may be selected from mixing, kneading, dissolution, immersion, scattering, spraying and application, for example, depending on the form of the preparation.

The content of the component (A) in the skin external preparation for cellulite improvement of the present invention is not particularly limited, but is preferably 0.01 to 10 mass%. In a case where the component (A) is α-glucosyl hesperidin, the content of α-glucosyl hesperidin is not particularly limited, but is preferably 0.01 to 10 mass%, more preferably 0.1 to 5 mass% and still more preferably 0.5 to 3 mass%. When the content is within the above-described range, the stability of α-glucosyl hesperidin is favorable, and cellulite is efficiently improved. In a case where the component (A) is α-glucosyl naringin, the content of α-glucosyl naringin is not particularly limited, but is preferably 0.01 to 10 mass%, more preferably 0.1 to 7 mass% and still more preferably 1 to 4 mass%. When the content is within the above-described range, the stability of α-glucosyl naringin is favorable, and cellulite is efficiently improved. In addition, in a case where the component (A) is α-glucosyl hesperidin and α-glucosyl naringin, the total content of α-glucosyl hesperidin and α-glucosyl naringin is not particularly limited, but is preferably 0.01 to 10 mass%, more preferably 0.1 to 8 mass% and still more preferably 0.5 to 6 mass%. When the content is within the above-described range, the stability of the component (A) is favorable, and cellulite is efficiently improved. Here, the amount of the skin external preparation for cellulite improvement is set to 100 mass%.

In a case where the component (A) is α-glucosyl hesperidin and α-glucosyl naringin, the amount ratio is not particularly limited, but the mass ratio between α-glucosyl hesperidin and α-glucosyl naringin is preferably 1:9 to 9:1 and more preferably 2:8 to 8:2.

Examples of the skin external preparation for cellulite improvement include medicines, quasi-drugs or cosmetic products such as emulsion, serum, lotion, cream, a mask, foundation, sunscreen, a cleaning agent, a makeup cosmetic, dispersion, ointment, a liquid agent, an aerosol, a patch, a poultice and a liniment. The skin external preparation for cellulite improvement may contain a variety of components such as water (e.g., purified water, hot spring water or deep sea water), alcohols, an oil agent, a surfactant, a metal soap, a gelator, a powder, a water-soluble polymer, a film-forming agent, a resin, an UV protection agent, an inclusion compound, an antibacterial agent, a fragrance, a deodorant, salts, a pH adjuster, a freshener, an animal/microorganism-derived extract, a plant extract, a stabilizer, an antioxidant, a blood circulation promoter, an astringent, an antiseborrheic drug, an anti-inflammatory agent, a cell activator, a moisturizer, a chelating agent, a keratolytic agent, an enzyme, hormones and vitamins to an extent that the effect of the present invention is not impaired as long as the components are normally blended with medicinal products, quasi-drugs or cosmetic products that are externally used for skin.

Regarding the skin external preparation for cellulite improvement, the component (A) is preferably administered to living bodies, particularly, human within a range of 0.001 to 10 mg/kg body weight per day. The amount to be administered is preferably 0.01 to 3 mg/kg body weight per day and more preferably 0.1 to 0.5 mg/kg body weight per day.

### <Moisturizer>

Skin external preparations for cellulite improvement that are externally used for skin may contain a moisturizer as an arbitrary component.

Examples of the moisturizer include polyhydric alcohols such as glycerin, diglycerin, glyceryl glucoside, ethylene glycol, diethylene glycol, propylene glycol, dipropylene glycol, 1,3-butylene glycol, 1,2-pentanediol, 1,2-hexanediol, polyethylene glycol and polypropylene glycol, sugars such as xylitol, maltitol, maltose, D-mannitol, glucose, fructose and trehalose, mucopolysaccharides such as sodium chondroitin sulfate and sodium hyaluronate, NMF components such as urea, sodium lactate and pyrrolidone sodium.

These moisturizers may be used singly or two or more moisturizers may be used in combination.

Among them, polyhydric alcohols are preferable and 1,3-butylene glycol or glycerin is more preferable from the viewpoint of high stability and the cellulite improvement effect. In a case where the skin external preparation for cellulite improvement of the present invention contains the moisturizer, the content of the moisturizer is preferably 1 to 30 mass% and more preferably 2 to 25 mass% per 100 mass% of the skin external preparation for cellulite improvement (composition).

### <Oil Agent>

Skin external preparations for cellulite improvement that are externally used for skin may contain an oil agent as an arbitrary component.

The nature of the oil agent does not matter, which means that the oil may be a natural oil or a synthetic oil and may be solid, semisolid or liquid, and examples thereof include synthetic ester oils that are fluid at 25°C under 1 atm such as isopropyl myristate, butyl myristate, isopropyl palmitate, ethyl oleate, ethyl linoleate, isopropyl linoleate, cetyl caprylate, hexyl laurate, decyl myristate, decyl oleate, oleyl oleate, isostearyl laurate, isotridecyl myristate, isocetyl myristate, isostearyl myristate, octyldodecyl myristate, octyl palmitate, isocetyl palmitate, isostearyl palmitate, isodecil oleate, isopropyl isostearate, cetyl 2-ethylhexanoate, stearyl 2-ethylhexanoate, propylene glycol isostearate, propylene glycol dicaprate, propylene glycol dioleate, glyceryl tri 2-ethylhexanoate, caprylic/capric triglyceride, isononyl isononanoate, diisopropyl sebacate, alkyl benzoate (C12 to C15) and ethylhexyl salicylate; hydrocarbons such as squalane, mineral oil (liquid paraffin) and vaseline; ester oils such as di-2-isooctadecyladipate, isononyl isononanoate, isotridecyl isononanoate, isodecyl isononanoate, cetyl isooctanoate, isostearyl isostearate, cetyl octanoate, oleyl oleate, ethyl oleate, decyl oleate, propylene glycol diisononanoate, neopentylene glycol di-2-ethylhexanoate, propylene glycol dicaprylate, ethylene glycol diisononanoate, neopentyl glycol dicaprate, isostearyl myristate, isopropyl myristate, isocetyl myristate, octyldodecyl myristate, isopropyl palmitate, isostearyl palmitate, 2-hexyldecyl palmitate, octyl palmitate, myristyl lactate, cetyl lactate, octyl lactate, diisostearyl malate, cholesteryl 12-hydroxystearate, pentaerythrityl tetraoctanoate, polyglyceryl tetraisostearate, pentaerythrityl tetra 2-ethylhexanoate, ethyl stearate, butyl stearate, ethyl linoleate, isopropyl linoleate, N-lauroyl-L-glutamic acid-2-octyldodecyl ester, isopropyl isostearate, neopentyl glycol dicaprate, decanoyl/octanoyl-glycerides, glyceryl tri 2-ethylhexanoate, trimethylolpropane trioctanoate, glyceryl trioctanoate, diglyceryl oligoester (2-hexyldecanoic acid/sevacinic acid), polypropylene glycol oligoester succinate, polyglyceryl triisostearate and polyglyceryl diisostearate; waxes such as beeswax, carnauba wax and candelilla wax; silicone oils such as dimethylpolysiloxane and methylphenylpolysiloxane; fluorine oils such as perfluoropolyether; oils and fats derived from plants or animals such as olive oil, castor oil, jojoba oil, macadamia nut oil, apricot kernel oil, persic oil, safflower oil, sunflower oil, avocado oil, medufoam oil, camellia oil, almond oil, perilla oil, sesame oil, borage oil and shea butter.

These oil agents may be used singly or two or more oil agents may be used in combination. In addition, among them, liquid-form oils are preferable, liquid-form hydrocarbons are more preferable, and mineral oil or squalane is still more preferable from the viewpoint of emulsion stability.

The content of the oil agent in the skin external preparation for cellulite improvement of the present invention is preferably 1 to 50 mass% and more preferably 5 to 30 mass% per 100 mass% of the skin external preparation for cellulite improvement (composition) from the viewpoint of the stability of the skin external preparation for cellulite improvement.

### <Surfactant>

Skin external preparations for cellulite improvement that are externally used for skin may contain a surfactant (emulsifier) as an arbitrary component.

The surfactant is not particularly limited as long as the surfactant is a surfactant that is normally used for medicinal products for skin external use and cosmetic products, and a non-ionic surfactant, an anionic surfactant and an ampholytic surfactant can be used, for example.

Examples of the non-ionic surfactant include silicone-based surfactants such as polyoxyalkylene-modified organopolysiloxane and polyoxyalkyl ether co-modified organopolysiloxane; polyethylene glycol dipolyhydroxy fatty acid esters such as dipolyhydroxystearate polyethylene glycol (EO: 2 to 40); glycerin fatty acid esters such as glyceryl stearate and alkylene glycol adducts thereof (e.g., PEG-15 glyceryl stearate); polyglycerin fatty acid esters and alkylene glycol adducts thereof; propylene glycol fatty acid esters and alkylene glycol adducts thereof; sorbitan fatty acid esters and alkylene glycol adducts thereof; fatty acid esters of sorbitol and alkylene glycol adducts thereof; polyalkylene glycol fatty acid ester; polyoxyalkylene alkyl ether; glycerin alkyl ether; polyoxyethylene alkyl phenyl ether; polyoxyethylene-cured castor oil; alkylene glycol adducts of lanolin; higher alcohols such as hexadecyl alcohol (cetanol) and lauryl alcohol.

Examples of the anionic surfactant include fatty acid soap such as N-stearoyl-N-methyltaurine sodium, sodium methyl myristoyl taurate, sodium polyoxyethylene cetylether phosphate, sodium polyoxyethylene lauryl ether phosphate and sodium stearate, sodium polyoxyethylene alkyl ether phosphate.

As the ampholytic surfactant, there are amino acid-type surfactants, betaine-type carboxylic acid type surfactants, sulfate ester-type surfactants, sulfonic acid-type surfactants and phosphate ester-type surfactants, and examples thereof include soybean phospholipid, N,N-dimethyl-N-alkyl-N-carboxymethyl ammonium betaine, N,N-dialkylaminoalkylenecarboxylic acid, N,N,N-trialkyl-N-sulfoalkylene ammonium betaine, N,N-dialkyl-N,N-bis(polyoxyethylene sulfate) ammonium betaine and 2-alkyl-1-hydroxyethyl-1-carboxymethylimidazolinium betaine.

These surfactants may be used singly or two or more surfactants may be used in combination. In addition, among them, non-ionic surfactants are preferable and glycerin fatty acid esters such as glyceryl stearate and alkylene glycol adducts thereof are more preferable from the viewpoint of emulsion stability.

The content of the surfactant in the skin external preparation for cellulite improvement of the present invention is preferably 0.5 to 20 mass% and more preferably 1 to 10 mass% per 100 mass% of the skin external preparation for cellulite improvement (composition) from the viewpoint of the stability of the skin external preparation for cellulite improvement.

### <Emulsifying Aid>

Skin external preparations for cellulite improvement that are externally used for skin may contain an emulsifying aid as an arbitrary component.

The emulsifying aid is not particularly limited as long as the emulsifying aid has an action of aiding emulsification by the surfactant, and examples thereof include fatty acids such as lauric acid, myristic acid, palmitic acid, stearic acid, linoleic acid, linolenic acid and oleic acid; higher alcohols such as stearyl alcohol and behenyl alcohol.

These emulsifying aids may be used singly or two or more emulsifying aids may be used in combination. In addition, among them, fatty acids and higher alcohols are preferable and stearic acid and behenyl alcohol are more preferable from the viewpoint of emulsion stability.

The content of the emulsifying aid in the skin external preparation for cellulite improvement of the present invention is preferably 0.1 to 15 mass% and more preferably 0.5 to 10 mass% per 100 mass% of the skin external preparation for cellulite improvement (composition) from the viewpoint of the stability of the skin external preparation for cellulite improvement.

### <Stabilizer>

Skin external preparations for cellulite improvement that are externally used for skin may contain an antioxidative stabilizer as an arbitrary component.

Specific examples of the stabilizer include ascorbyl tetraisopalmitate, ascorbyl stearate, ascorbyl palmitate, tocopherols, rosemary extracts, tea extracts, dibutylhydroxytoluene, butylated hydroxyanisole and phenolic antioxidants.

These stabilizers may be used singly or two or more stabilizers may be used in combination.

Among them, tocopherol and tocopheryl acetate are preferable since the oxidative stability is high and the influence on fragrance and color at the time of being used to produce preparations is small. The content of the stabilizer in the skin external preparation for cellulite improvement of the present invention is preferably 0.0001 to 3 mass% and more preferably 0.001 to 1 mass% per 100 mass% of the skin external preparation for cellulite improvement (composition).

### <Preservative>

Skin external preparations for cellulite improvement that are externally used for skin may contain a preservative as an arbitrary component.

Examples of the preservative include benzoic acid, benzoates, alkyldiaminoethylglycine hydrochloride, a bioactive dye, chlorocresol, chlorobutanol, salicylic acid, salicylates, sorbic acid and salts thereof, dehydroacetic acid and salts thereof, trichlorohydroxydiphenyl ether (also known as triclosan), p-hydroxybenzoate ester (paraben) and sodium salts thereof, phenoxyethanol, phenethyl alcohol, phenol, sodium lauryl diethylenediaminoglycinate, resorcin, zinc/ammonia/silver composite-substituted zeolite, panthenyl ethyl ether benzoate, isopropyl methylphenol, cetylpyridinium chloride, benzalkonium chloride, benzethonium chloride, chlorhexidine hydrochloride, orthophenylphenol, sodium orthophenyl phenol, silver-copper zeolite, chlorhexidine gluconate, cresol, chloramine T, chloroxylenol, chlorphenesin, chlorhexidine, 1,3-dimethylol-5,5-dimethylhydantoin, lauryl isoquinolinium bromide, thianthol, thymol, trichlorocarbanilide, parachlorphenol, halocarban, hinokitiol, zinc pyrithione, piroctone olamine, iodopropynyl butylcarbamate, polyaminopropyl biguanide, methylisothiazolinone, a methylchloroisothiazolinone/methylisothiazolinone solution, N,N'-methylenebis[N'-(3-hydroxymethyl-2,5-dioxo-4-imidazolidinyl) urea] and p-dimethylaminostyryl heptyl methyl triazolium iodide.

These preservatives may be used singly or two or more preservatives may be used in combination.

Among them, phenoxyethanol, ethyl paraoxybenzoate and methyl paraoxybenzoate are preferable since the effect as a preservative is high and the influence on fragrance and color at the time of being used to produce preparations is small. The content of the preservative in the skin external preparation for cellulite improvement of the present invention is preferably 0.001 to 3 mass% and more preferably 0.01 to 1 mass% per 100 mass% of the skin external preparation for cellulite improvement (composition).

### <Cellulite Improvement Method>

A cellulite improvement method of the present invention needs to include a step of externally applying a skin external preparation for cellulite improvement containing at least one component (A) selected from α-glucosyl hesperidin and α-glucosyl naringin to the skin of a target.

As the skin external preparation for cellulite improvement that is used in the cellulite improvement method of the present invention, the above-described skin external preparation for cellulite improvement of the present invention is used.

The method for externally applying the skin external preparation for cellulite improvement to the skin of a target is not particularly limited, and an arbitrary preferable method can be used depending on the target. Examples thereof include a simple application method, multilayer dressing, occlusive dressing and hydrocolloid dressing.

In order to efficiently improve cellulite, the cellulite improvement method of the present invention preferably includes a step of massaging skin in at least one timing of before the step of externally applying the skin external preparation for cellulite improvement for the skin, at the same time as the step of externally applying the skin external preparation for cellulite improvement for the skin and after the step of externally applying the skin external preparation for cellulite improvement for the skin. The step of massaging skin is preferably included after the step of externally applying the skin external preparation for cellulite improvement for the skin.

In order to efficiently improve cellulite, in the cellulite improvement method, the component (A) is preferably administered 0.001 to 10 mg/kg body weight per day. The amount to be administered is preferably 0.01 to 3 mg/kg body weight per day and more preferably 0.1 to 0.5 mg/kg body weight per day.

The cellulite improvement method of the present invention preferably has a step of evaluating the cellulite of a target by ultrasonic tomography.

### Examples

Next, the present invention will be described in more detail by showing Examples, but the present invention is not limited to these Examples.

### [Example 1] Human study by application of test sample

### <<Method>>

8 subjects were asked to apply α-glucosyl hesperidin-blended massage cream or α-glucosyl naringin-blended massage cream to their own bodies, and changes in cellulite were investigated.

### [Study schedule]

As the study, an open trial was carried out, and the measurement on the subjects were conducted before the initiation of the test, after 4 weeks of the application and after 8 weeks of the application.

### [Subjects]

Persons that satisfied all of the following entry criteria and did not satisfy any of exclusion criteria were selected as the subjects. The ages of the 8 subjects were 38.3 ± 8.2 years old (average ± standard deviation)

Entry criterion 1: Japanese women aged 30 years or more and 49 years or less when consent is obtained

Entry criterion 2: Persons having cellulite on the back sides of the thighs (persons from which it is possible to confirm that the flesh on the back sides of the thighs become bumpy when grasped and twisted as if a dustcloth is squeezed)

Entry criterion 3: Persons having BMI of 22 or more and 35 or less

Exclusion criterion 1: Persons who are pregnant or possibly pregnant or are breast-feeding

Exclusion criterion 2: Persons who may show allergic symptoms due to the component of a test sample

Exclusion criterion 3: Persons who participate in other clinical studies

The study was carried out after written consent to the participation to the study was obtained on a voluntary basis based on ethical considerations according to Declaration of Helsinki.

### [Test sample]

As the test sample, α-glucosyl hesperidin-blended massage cream or α-glucosyl naringin-blended massage cream was used. The composition of the present test sample is as described below. "%" indicates "mass%".

(α-glucosyl hesperidin-blended massage cream: hereinafter, also referred to as citrus-derived polyphenol-containing cream HES or cream containing 1% αG hesperidin PS-CC)

| | |
|---|---|
| αG Hesperidin PS-CC (manufactured by Toyo Sugar Refining Co., Ltd.) | 1.0% |
| Mineral oil | 20.0% |
| Behenyl alcohol | 5.0% |
| Stearic acid | 1.0% |
| Glyceryl stearate | 2.5% |
| PEG-15 glyceryl stearate | 2.0% |
| Methylparaben | 0.2% |
| Phenoxyethanol | 0.6% |
| Tocopherol | 0.05% |
| 1,3-Butylene glycol | 4.0% |
| Glycerin | 1.0% |
| Water | 62.65% |
| Total | 100.0% |

(α-glucosyl naringin-blended massage cream: hereinafter, also referred to as citrus-derived polyphenol-containing cream NAR or cream containing 2% αG naringin)

| | |
|---|---|
| αG naringin (third enzyme-treated naringin) | 2.0% |
| Mineral oil | 20.0% |
| Behenyl alcohol | 5.0% |
| Stearic acid | 1.0% |
| Glyceryl stearate | 2.5% |
| PEG-15 glyceryl stearate | 2.0% |
| Methylparaben | 0.2% |
| Phenoxyethanol | 0.6% |
| Tocopherol | 0.05% |
| 1,3-Butylene glycol | 4.0% |
| Glycerin | 1.0% |
| Water | 61.65% |
| Total | 100.0% |

### (Production Method)

Oily components heated to 80°C were stirred, aqueous components heated to 80°C were added thereto to emulsify, and αG hesperidin PS-CC or αG naringin was added thereto and mixed therewith. The produced creams were individually loaded into jars, "application on the right-hand side" was noted on the jars containing α-glucosyl hesperidin-blended massage cream, and "application on the left-hand side" was noted on the jars containing α-glucosyl naringin-blended massage cream.

### [Application]

The cream was applied twice a day (morning and night). The application in the morning was carried out after waking up or during daytime, and the application at night was carried out after a bath. The amount of the cream used was set to an amount of the cream taken using the index finger from the tip to the first joint (approximately 0.5 g/application). In a case where the amount of the cream was not enough for the application, the amount of the cream applied was allowed to be increased up to three times.

### (Application Order)

1. The test sample designated as "application on the right-hand side" was applied to the right leg. An amount of the test sample taken using the index finger from the tip to the first joint (approximately 0.5 g/application) was moved to the palm of the hand, and the test sample was applied to the back of the knee of the right leg through the hip while massaging the skin as if the skin was pulled up from down.

2. Once the application to the right leg ended, the hands were washed.

3. The test sample designated as "application on the left-hand side" was applied to the left leg. An amount of the test sample taken using the index finger from the tip to the first joint (approximately 0.5 g/application) was moved to the palm of the hand, and the test sample was applied to the back of the knee of the left leg through the hip while massaging the skin as if the skin was pulled up from down.

### [Evaluation]

### (Measurement of Thigh Circumference Length)

A site 10 cm below the crotch of the subject was measured with a handy 3D scanner (non-contact method/three-dimensional human body measurement system, manufactured by Altech Co., Ltd.). At the time of the first observation, 10 cm from the crotch was measured with a ruler, the distance of that site from the floor was also measured, and, in the second and later observations, the distance from the floor was regarded as the same, thereby observing the site of the thigh at the same height at every observation. In a single round of observation, the measurement was carried out three times, and the median was employed.

### (Cellulite Improvement Effect)

A site 10 cm below the crotch of the subject on the back side of the thigh was measured with an ultrasonic tomography device DermaLab (manufactured by Cortex Technology). At the time of the first observation, 10 cm from the crotch was measured with a ruler, the distance of that site from the floor was also measured, and, in the second and later observations, the distance from the floor was regarded as the same, thereby observing the site of the thigh at the same height at every observation. In addition, the width of the back side of the thigh was measured at the same height with a ruler, and the central portion of a measurement probe was put on the center of the width.

The measurement was carried out by scanning 3/4 of the circumference of a circle with a diameter of 7.5 mm (17.6 mm) for one second while ultrasonic waves were emitted from a piezoelectric element. The measurement width was 150 µm, the measurement depth was 0.7556 mm, and the number of pixels was 256 x 393.

The intensity of reflected sound was captured as an image by dividing the individual pixels into 100 tones and classifying the pixels into five colors of black, green, red, yellow and white from the low tone. The image was displayed in a manner of being enlarged 4.5 times in the depth direction. The values of the cellulite area, the collagen score, the age band and the thickness were calculated from the obtained image by the following methods. In a single round of observation, the measurement was carried out three times, and, as the values of the values of the cellulite area, the collagen score, the age band and the thickness, the medians of values calculated from three images, which were each obtained from each measurement, were employed.

### (Cellulite Area)

The cellulite area represents the disorder of the boundary surface between the dermis and the subcutaneous tissue and was calculated with a program built in DermaLab. Specifically, the cellulite area was calculated by the following method. A green pixel present at the deepest part of the skin was selected, and a straight line A parallel to the skin surface was drawn at the depth of the pixel. A boundary line B between green and black was drawn in a direction shallower than the straight line A. The area of a portion surrounded by the straight line A and the boundary line B was regarded as the cellulite area. In a case where the ends of the boundary line B were not in contact with the straight line A, perpendicular lines C were drawn from the ends of the boundary line B to the straight line A, and the area of a portion surrounded by the straight line A, the boundary line B and the perpendicular lines C was regarded as the cellulite area.

### (Collagen Score)

The collagen score represents the density of collagen in the dermis and was calculated with the program built in DermaLab.

### (Age Band)

The age band is a part with less collagen that is formed right below the epidermis due to a decrease in collagen. The width (µm) of the age band was calculated with the program built in DermaLab.

### (Thickness)

The thickness refers to the thickness (µm) of the dermis collagen layer and was calculated with the program built in DermaLab.

### <<Result>>

### (Thigh Circumference Length)

The measurement results of the thigh circumference lengths are shown in Table 1.

### [Table 1]

**Table 1**

| Item | Unit | Before application | After 4 weeks of application | Temporal P value | After 8 weeks of application | Temporal P value |
|---|---|---|---|---|---|---|
| Circumference length (right thigh), application of citrus-derived polyphenol-containing cream HES | **mm** | **528.3 ± 24.6** | **526.2 ± 30.8** | ***P* = 0.720** | **526.2 ± 31.3** | ***P* = 0.723** |
| Circumference length (left thigh), application of citrus-derived polyphenol-containing cream NAR | **mm** | **526.5 ± 14.8** | **523.5 ± 20.4** | ***P* = 0.512** | **525.5 ± 23.1** | ***P* = 0.913** |

| | | | | | | |
|---|---|---|---|---|---|---|
| Average ± standard deviation **n=8** | | | | | | |

No significant changes in thigh circumference length were observed before and after the application of the α-glucosyl hesperidin- or α-glucosyl naringin-blended massage cream.

### (Cellulite Improvement Effect)

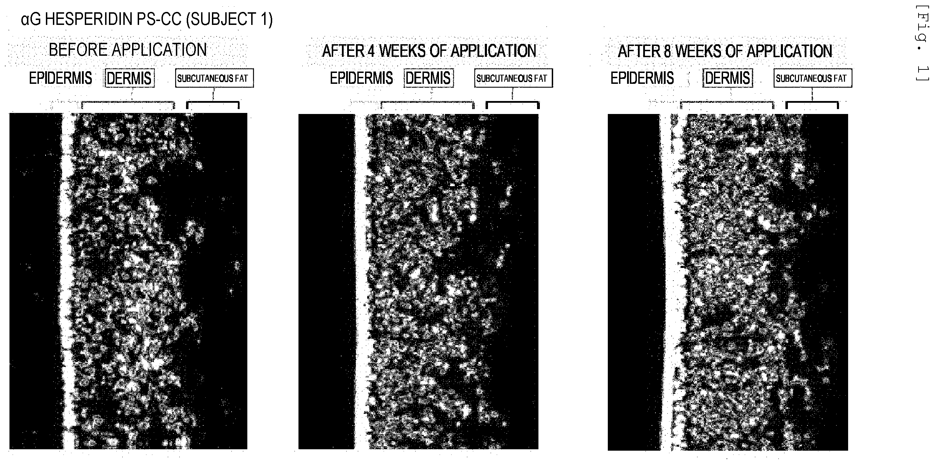

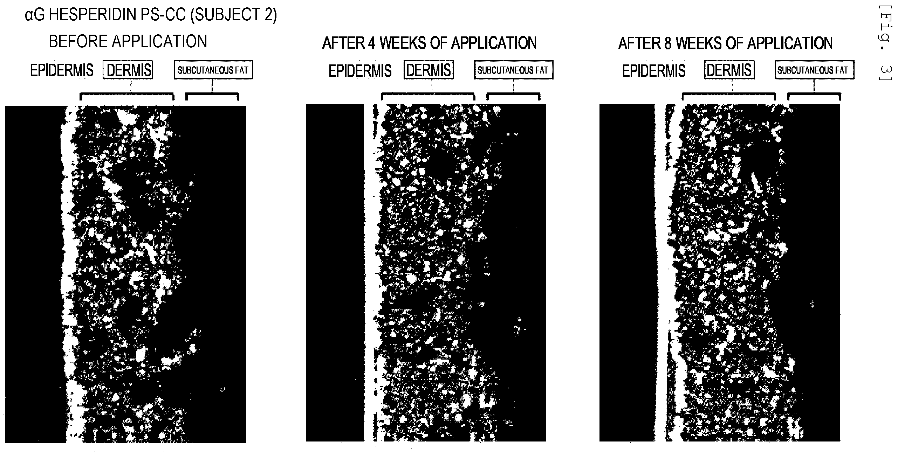

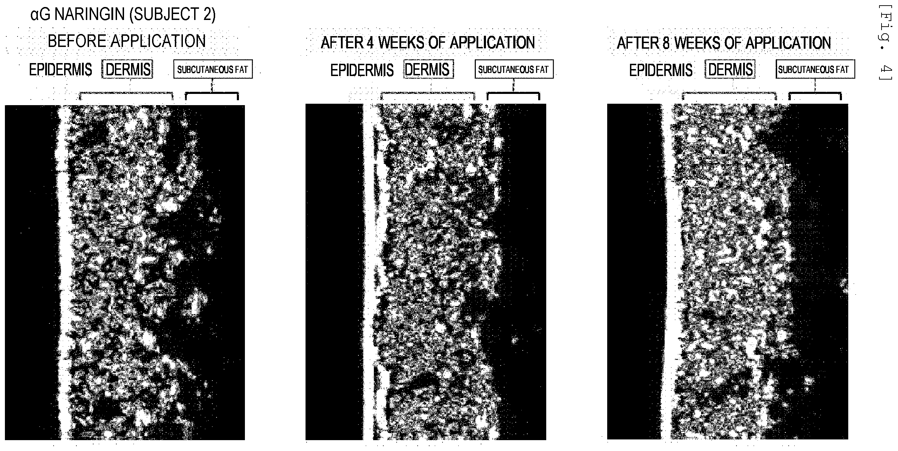

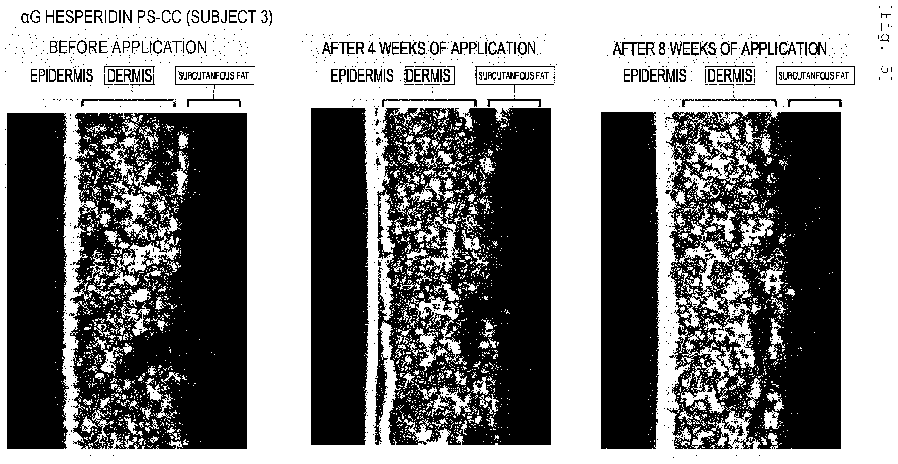

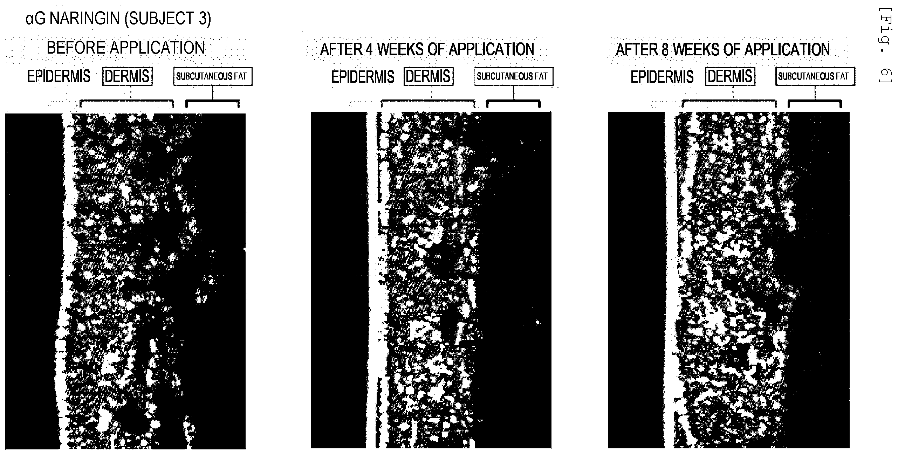

Images of the back sides of the thighs captured with the ultrasonic tomography device before the application of the α-glucosyl hesperidin-blended massage cream, after 4 weeks of the application and after 8 weeks of the application are shown in Figure 1 to Figure 6. The images are equivalent to 17.6 mm in length and 3.4 mm in width. According to the density of collagen, these images are displayed in black, green, red, yellow and white from the low density. Since collagen was abundantly contained in the dermis, but not contained in the subcutaneous fat, in normal skin, the dermis is displayed in green, red, yellow or white, and the subcutaneous fat is displayed in black. In addition, the epidermis is also a hyper echo tissue and thus displayed in white. On the other hand, in skin containing cellulite, normally, there are black portions even in the dermis, and the boundary line between black and green waves between the dermis and the subcutaneous fat.

In Figure 1, before the application of the test sample, there are black portions even in the dermis, and the boundary line between black and green waves between the dermis and the subcutaneous fat. In subject 1, the black portions in the dermis decreased after 4 weeks of the application of the α-glucosyl hesperidin-blended massage cream and after 8 weeks of the application, and, in particular, after 8 weeks, the portion where the boundary line between black and green waved decreased, and the dermis and the subcutaneous fat were divided almost linearly. That is, it is assumed that, in the skin of the site to which the α-glucosyl hesperidin-blended massage cream was applied, cellulite was improved.

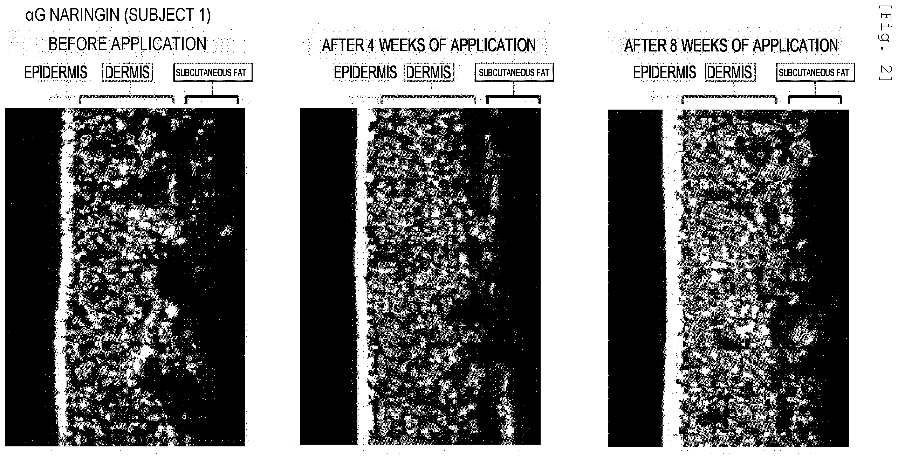

Images of the back side of the thigh of subject 1 captured with the ultrasonic tomography device before the application of the α-glucosyl naringin-blended massage cream, after 4 weeks of the application and after 8 weeks of the application are shown in Figure 2.

Before the application, there are black portions even in the dermis, and the boundary line between black and green waves between the dermis and the subcutaneous fat. The black portions in the dermis decreased after 4 weeks of the application of the α-glucosyl naringin-blended massage cream and after 8 weeks of the application, and, in particular, after 8 weeks, the portion where the boundary line between black and green waved decreased, and the dermis and the subcutaneous fat were divided almost linearly. That is, it is assumed that, in the skin of the site to which the α-glucosyl naringin-blended massage cream was applied, cellulite was improved.

In cases where the α-glucosyl hesperidin-blended massage cream or the α-glucosyl naringin-blended massage cream was applied to subject 2 and subject 3 as well, the same results as for subject 1 were obtained.

The values of the cellulite area, the collagen score, the age band and the thickness are shown in Table 2.

### [Table 2]

**Table 2**

| Item | Unit | Before application | After 4 weeks of application | Temporal P value | After 8 weeKs of application | Temporal P value |
|---|---|---|---|---|---|---|
| Colloagen Score (back side of right thigh), application of citrus-derived polyphenol-containing cream HES | **-** | **40.5 ± 9.9** | **47.9 ± 9.3** | ***P* = 0.080** | **58.5 ± 11.1** | **P < 0.001 ^{∗∗∗}** |
| Age band (back side of right thigh), application of citrus-derived polyphenol-containing cream HES | **µm** | **93.8 ± 83.9** | **16.5 ± 28.3** | ***P* = 0.008 ^{∗∗}** | **10.5 ± 15.8** | ***P* = 0.005 ^{∗∗}** |
| Thickness (back side of right thigh), application of citrus-derived polyphenol-containing cream HES | **-** | **1482 ± 428** | **1106 ± 151** | ***P* = 0.030 ^{∗}** | **1141 ± 177** | ***P* = 0.048 ^{∗}** |
| Cellulite (area) (back side of right thigh), application of citrus-derived polyphenol-containing cream HES | **-** | **256.0 ± 50.3** | **197.6 ± 61.6** | ***P* = 0.013 ^{∗}** | **167.9 ± 49.4** | ***P* < 0.001 ^{∗∗∗}** |
| Colloagen Score (back side of left thigh), application of citrus-derived polyphenol-containing cream NAR | **-** | **39.6 ± 7.3** | **46.4 ± 10.1** | ***P* = 0.041 ^{∗}** | **57.6 ± 9.9** | ***P* < 0.001 ^{∗∗∗}** |
| Age band (back side of left thigh), application of citrus-derived polyphenol-containing cream NAR | **µm** | **98.1 ± 94.8** | **20.3 ± 27.6** | ***P* = 0.011 ^{∗}** | **21.4 ± 58.7** | ***P* = 0.012 *** |
| Thickness (back side of left thigh), application of citrus-derived polyphenol-containing cream NAR | **-** | **1378 ± 249** | **1228 ± 176** | ***P* = 0.120** | **1252 ± 124** | ***P* = 0.204** |
| Cellulite (area) (back side of left thigh), application of citrus-derived polyphenol-containing cream NAR | **-** | **288.2 ± 89.6** | **186.0 ± 96.1** | ***P* = 0.004 ^{∗∗}** | **148.7 ± 52.9** | ***P* < 0.001 ^{∗∗∗}** |

| | | | | | | |
|---|---|---|---|---|---|---|
| Average ± standard deviation n=8 * Statistical analysis was carried out by Dunnett's test for ^{∗}:P<0.05^{∗∗}:P<0.01 ^{∗∗∗}:P<0.001 before application and after 4 weeks of application and for before application and after 8 weeks of application. | | | | | | |

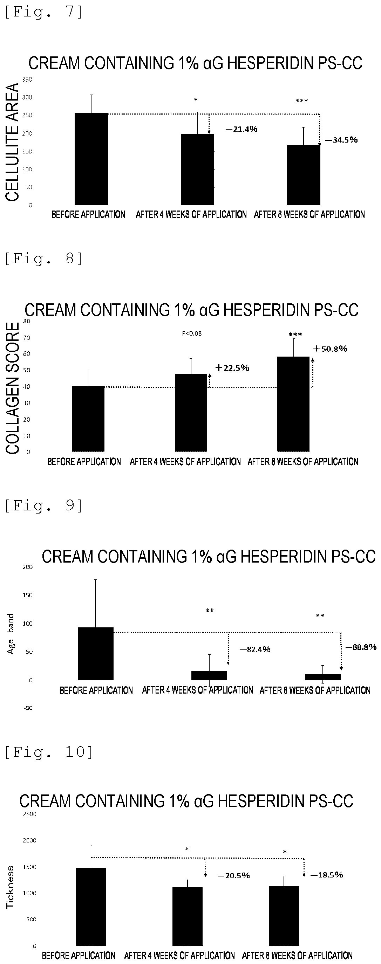

Figure 7 is a graph of the cellulite areas in a case where the α-glucosyl hesperidin-blended massage cream was applied, which is based on Table 2. The cellulite area decreased due to the application, and significant differences were observed after 4 weeks of the application and after 8 weeks. Figure 8 is a graph of the collagen scores in a case where the α-glucosyl hesperidin-blended massage cream was applied, which is based on Table 2. The collagen score increased due to the application, and a significant difference was observed after 8 weeks of the application. Figure 9 is a graph of the age bands in a case where the α-glucosyl hesperidin-blended massage cream was applied, which is based on Table 2. The age band decreased due to the application, and significant differences were observed after 4 weeks of the application and after 8 weeks. Figure 10 is a graph of the thicknesses in a case where the α-glucosyl hesperidin-blended massage cream was applied, which is based on Table 2. The thickness decreased due to the application, and significant differences were observed after 4 weeks of the application and after 8 weeks.

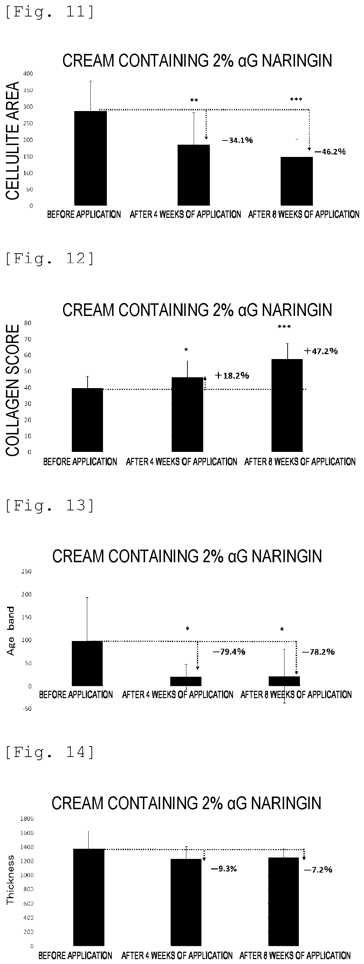

Figure 11 is a graph of the cellulite areas in a case where the α-glucosyl naringin-blended massage cream was applied, which is based on Table 2. The cellulite area decreased due to the application, and significant differences were observed after 4 weeks of the application and after 8 weeks. Figure 12 is a graph of the collagen scores in a case where the α-glucosyl naringin-blended massage cream was applied, which is based on Table 2. The collagen score increased due to the application, and significant differences were observed after 4 weeks of the application and after 8 weeks of the application. Figure 13 is a graph of the age bands in a case where the α-glucosyl naringin-blended massage cream was applied, which is based on Table 2. The age band decreased due to the application, and significant differences were observed after 4 weeks of the application and after 8 weeks. Figure 14 is a graph of the thicknesses in a case where the α-glucosyl naringin-blended massage cream was applied, which is based on Table 2. No significant difference was observed, but there was a tendency that the thickness decreased due to the application.

Due to the use of the α-glucosyl hesperidin-blended massage cream or the α-glucosyl naringin-blended massage cream, cellulite improvement was observed in the evaluation by the ultrasonic tomographic capturing although the circumference lengths of the thighs were not changed. Therefore, it is considered that, due to α-glucosyl hesperidin or α-glucosyl naringin, the state of connective tissues changes and cellulite improves.

### Industrial Applicability

According to the present invention, it is possible to provide a skin external preparation for cellulite improvement and a cellulite improvement method that do not require any special equipment and manipulation and are capable of conveniently improving cellulite.

## Claims

1. A skin external preparation for cellulite improvement, comprising at least one component (A) selected from α-glucosyl hesperidin and α-glucosyl naringin.

2. The skin external preparation for cellulite improvement according to claim 1, being for massage.

3. The skin external preparation for cellulite improvement according to claim 1 or 2, wherein a content of the component (A) is 0.01 to 10 mass%.

4. A cellulite improvement method, comprising a step of externally applying the skin external preparation for cellulite improvement according to any one of claims 1 to 3 to the skin of a target.

5. The cellulite improvement method according to claim 4, further comprising a step of massaging the skin in at least one timing of before the step of externally applying the skin external preparation for cellulite improvement for the skin, at the same time as the step of externally applying the skin external preparation for cellulite improvement for the skin and after the step of externally applying the skin external preparation for cellulite improvement for the skin.

6. The cellulite improvement method according to claim 4 or 5, wherein the component (A) is administered at 0.001 to 10 mg/kg body weight per day.

7. The cellulite improvement method according to any one of claims 4 to 6, further comprising a step of evaluating the cellulite of a target by ultrasonic tomography.
